# EUROPÄISCHE PATENTSCHRIFT

(11) **EP 0 863 750 B1**
(45) Veröffentlichungstag und Bekanntmachung des Hinweises auf die Patenterteilung: **03.09.2003**
(21) Anmeldenummer: 96940659.4
(22) Anmeldetag: 29.11.1996
(51) Int. Cl.: A61K 9/48

(54) **PHARMAZEUTISCHE PRÄPARATE ZUR ORALEN ANWENDUNG UND VERFAHREN ZU IHRER HERSTELLUNG**
PHARMACEUTICAL PREPARATIONS FOR ORAL ADMINISTRATION AND PROCESS FOR THEIR PRODUCTION
PREPARATIONS PHARMACEUTIQUES POUR ADMINISTRATION PAR VOIE ORALE, ET LEUR PROCEDE DE PRODUCTION

(30) Priorität: 02.12.1995 DE 19545043
(43) Veröffentlichungstag der Anmeldung: 16.09.1998
(73) Patentinhaber: R.P. Scherer GmbH, 69402 Eberbach (DE)
(72) Erfinder: LUCKS, Jörn-Stefan, D-69412 Eberbach (DE); FISCHER, Gerhard, D-69412 Eberbach (DE)
(74) Vertreter: Werner, Hans-Karsten, Dr.Dipl.-Chem.
(86) Internationale Anmeldenummer: EP9605281
(87) Internationale Veröffentlichungsnummer: WO97020548

(56) Entgegenhaltungen:
- EP-A- 0 517 412

## Beschreibung

Flüssigbefüllte Gelatinekapseln zur oralen Anwendung werden entweder als Weichgelatinekapseln nach dem Rotary-Die-Verfahren oder als Hartgelatinekapseln in der Form hergestellt, daß das Kapselfüllgut über eine geeignete Dosiereinrichtung in die vorgefertigten leeren Kapseln eindosiert wird. Die Wahl des für den Wirkstoff jeweils bestgeeigneten Trägermediums richtet sich nach der Unbedenklichkeit, der technischen Verarbeitungsfähigkeit, der physikalischen und chemischen Stabilität der Kapseln und der Wirksamkeit des Endproduktes.

Für die Wirksamkeit im Endprodukt ist es wichtig, daß die Wirkstoffe nach der Einnahme die bestmögliche Bioverfügbarkeit aufweisen. Um eine gute Bioverfügbarkeit zu gewährleisten, haben sich einphasige Systeme bewährt, in denen der Wirkstoff gelöst ist und die bei Kontakt mit den gastrointestinalen Flüssigkeiten zu keinen spontanen Ausfällungen des Wirkstoffes führen.

So beschreibt die EP-A-0 257 386 ein Gemisch aus mindestens 5 % Ethanol und mindestens 20 % Partialglyceriden. Der Erfindung liegt die Beobachtung zugrunde, daß Ethanol als Lösungsmittel in ausreichender Konzentration nur dann in Gelatinekapseln abgefüllt werden kann, wenn das Cosolvents eine hinreichend hohe Konzentration an Partialglyceriden zugesetzt werden. Wie die Beispiele der Anmeldung zeigen, ist es zwar möglich physikalisch stabile Kapseln zu erhalten. Ein erheblicher Verlust an Ethanol im Füllgut ist aber immer vorhanden, der dazu zwingt Verdunstungsverluste an Ethanol vor der Verkapselung und migrationsbedingte Verluste bei der Trocknung durch entsprechende Produktionszuschläge auszugleichen.

EP-A-0 517 412 beschreibt pharmazeutische Zusammensetzungen umfassend Benzodiazepine zusammen mit pharmazeutisch verträglichen Ölen und pharmazeutisch verträglichen Tensiden sowie gegebenenfalls Weichmachern, Wasser und α-D-tocopherol.

Die EP-A-0 152 945 beschreibt pharmazeutische Mehrkomponentensysteme aus bis zu 70 % Öl, 2 bis 60 % Emulgator, bis zu 78 % Co-Emulgator und gegebenenfalls bis zu 85 % Wasser. Der Unterschied zwischen Emulgatoren und Co-Emulgatoren bzw. Tensiden und Co-Tensiden ist der sogenannte HLB-Wert, der über oder unter 8 liegt; vgl. Seite 5, Zeilen 13 bis 15. Das beschriebene System enthält Wasser und eine Reihe von Hilfsstoffen, die in erster Linie für die dermale Anwendung geeignet sind. Das Vorhandensein von Wasser schränkt die Löslichkeit des Wirstoffs in der Formulierung ein. Weiterhin verursacht Wasser Wechselwirkungen mit der Kapselhülle und ist daher zur Formulierung von Kapseln ungeeignet.

Die DE 39 27 113 A1 beschreibt ein Mittel, welches zwei schwerlösliche Wirkstoffe,nämlich Ibuprofen und Codein nur teilweise gelöst, aber teilweise suspendiert in einem System enthält, welches ausschließlich aus Tensiden besteht mit einem HLB-Wert größer als 8. Dieses System bereitet bei der Verkapselung in Weichgelatinekapseln Probleme, die durch den hohen Anteil von ungelösten Wirkstoffen etwas vermindert werden. Es handelt sich um ein mehrphasiges System, welches obendrein keine Co-Tenside enthält.

Die DE 36 11 467 A1 enthält den Wirkstoff Hymecromon teils gelöst, teils in nicht kristalliner Form suspendiert in einem Gemisch aus Polyalkylenglycol, einem Tensid und Propandiol. Es handelt sich um ein mehrphasiges System.

Die DD 298 351 A5 beschreibt ein Verfahren zur Herstellung einer carrierabhängigen Arzneiform, bei der der Wirkstoff in einen niederen mehrwertigen Alkohol C3 bis C6 gelöst wird, dann gemeinsam mit 1 bis 3 Carriersubstanzen in eine Schmelzphase übergeführt wird und nach dem Prinzip der Liquidabfüllung in Gelatinekapseln eingebracht wird, wobei die Schmelzerstarrung mit einer gesättigten organischen Säure durchgeführt wird. Sofern Tenside verwendet werden, handelt es sich vorzugsweise um solche mit einem HLB-Wert zwischen 10 und 50; vgl. Anspruch 3. Es handelt sich um ein System, das für Weichgelatinekapseln nicht geeignet ist.

Die US 5,281,420 beschreibt eine feste Dispersion des Wirkstoffes Tebufelone in einem Tensid. Vorzugsweise soll dabei der HLB-Wert größer als 14 sein; vgl. Anspruch 4. Es handelt sich um ein mehrphasiges System, das obendrein nur für Gelatinekapseln geeignet ist.

Die WO 94/23733 beschreibt eine pharmazeutische Zubereitung zur oralen Verabreichung, enthaltend Ciclosporin als Wirkstoff und einen Alkylenpolyether oder -ester, wobei der HLB-Wert der eingesetzten Komponente B mindestens 10 betragen muß. Diese Zubereitung wird gemäß Anspruch 7 vorzugsweise als Hartgelatinekapseln oder in Form einer Tablette hergestellt. Dies ist darauf zurückzuführen, daß Versuche, diese Rezepturen in Weichgelatinekapseln einzubringen, gescheitert sind.

Die DE-A-43 22 836 beschreibt eine Trägerzusammensetzung aus einem oder mehreren Polyglycerinfettsäureestern oder Sorbitan fettsäureestern, einem pharmazeutisch gebräuchlichen Öl und einem nicht-ionischen Tensid mit einem HLB Wert von mindestens 10 für die Formulierung von schwer wasserlöslichen Wirkstoffen. Konzentrationen von 20 bis 45 % nicht-ionischer Tenside mit einem HLB Wert von mindestens 10 werden bevorzugt. In den Beispielen 2 und 3 werden sogar mehr als 50 % Tensidkonzentration beschrieben. In Sucker, Fuchs, Speiser: Pharmazeutische Technologie, zweite Auflage, Seite 342, ist beschrieben, daß höhere Anteile von Tensiden erhöhte verarbeitungstechnische Probleme bei der Kapselnahtbildung mit sich bringen. Dadurch kommt es zu einem erhöhten Ausschuß durch offene Kapseln, so daß derartig hohe Tensidkonzentrationen für die Formulierung von Kapseln nicht in Frage kommen.

Aufgabe der vorliegenden Erfindung ist daher das Zurverfügung stellen eines Trägersystems *für* Arzneistoffe, welches auf den Zusatz größerer Mengen von Tensiden verzichtet, die bekanntlich die Nahtbildung von Weichgelatinekapseln stören, wobei ohne den leicht verdunstenden Träger Ethanol gearbeitet werden soll. Primär soll auch möglichst wenig oder kein Wasser enthalten sein, welches mit der Kapselhülle in Wechselwirkung treten kann. Dennoch soll dieses Trägersystem in hohe Lösekraft für pharmazeutische Wirkstoffe aufweisen und beim Vermischen mit Wasser keine spontane Rekristallisation des Wirkstoffes zulassen.

Diese Aufgabe kann jetzt überraschend einfach gelöst werden dadurch, daß der Wirkstoff gelöst ist in einem einphasigen System enthaltend jeweils bezogen auf das wirkstoffreie System 1 bis 20 Gew.-% Polyethylenglycole und/oder Benzylalkohol, 1 bis 20 Gew.-% eines oder mehrerer Tenside, 75 bis 98 Gew.-% eines oder mehrerer Co-Tenside und weniger als 5 Gew.-% Ethanol und weniger als 10 Gew.-% Wasser.

Als Polyethylenglycole werden bevorzugt eingesetzt die pharmazeutisch brauchbaren und unbedenklichen Polyethylenglycole mit einem Molekulargewicht von 300 bis 1.000. Vorzugsweise werden sie in Mengen zwichen 2,5 und 12,5 Gew.-% bezogen auf die wirkstofffreie Lösung eingesetzt. Weiterhin können diese Polyole ganz oder teilweise durch Benzylalkohol ersetzt werden.

Als Tenside werden vorzugsweise eingesetzt Blockpolymere von Polyoxiethylen und Polyoxipropylen, wie z.B. Pluronics^{R}, Polyoxyricinolfettsäureester, wie z.B. Cremophor^{R}, Polysorbate, wie z.B. Tween^{R}, Polyoxiethylenfettsäureester, wie z.B. Myrj^{R}, Polyoxiethylenglycerinmonoester, wie z.B. verschiedene Typen aus der Tagat^{R}-Reihe. Diese Tenside werden vorzugsweise in Mengen von 5 bis 15 Gew.-% bezogen auf die wirkstofffreie Mischung eingesetzt.

Als Co-Tenside werden vorzugsweise eingesetzt Partialglycoride von gesättigten oder ungesättigten Fettsäuren mit einer Kettenlänge von C6 bis C20. Derartige Produkte werden unter den Warenzeichen Inwitor^{R}, Softigen^{R}, Rilanit^{R}, Tegomuls^{R} und Capmul^{R} vertrieben. Weiterhin sind geeignet acetylierte Partialglyceride, wie z.B. Myracet^{R}, und Sorbitanfettsäureester, wie z.B. Produkte aus der Span^{R}-Reihe. Diese Substanzen werden vorzugsweise in Mengen von 79 bis 90 Gew.-% eingesetzt.

Auf den Zusatz von Ethanol und/oder Wasser wird vorzugsweise ganz verzichtet. Dennoch sind geringe Mengen in diese Lösungsmittel zulässig, ohne das gute Ergebnis zu gefährden. Auf alle Fälle sollten die Mengen dieser Lösungsmittel nur unter 5 Gew.-% bzw. 10 Gew.-% bezogen auf das einphasige System vorhanden sein.

Das erfindungsgemäße Verfahren zur Herstellung von pharmazeutischen Präparaten zur oralen Anwendung in flüssigbefüllten Weichgelatinekapseln ist dadurch gekennzeichnet, daß der Wirkstoff gelöst wird in einem einphasigen System, enthaltend jeweils bezogen auf das wirkstoffreie System 1 bis 20 Gew.-% pharmazeutisch verträgliche Polyole und/oder Benzylalkohol, 1 bis 20 Gew.-% eines oder mehrerer. Tenside, 75 bis 98 Gew.-% eines oder mehrerer Co-Tenside und weniger als 5 Gew.-% Ethanol und weniger als 10 Gew.-% Wasser, und in an sich bekannter Weise in Gelatinekapseln abgefüllt wird.

Vorzugsweise wird dieses System hergestellt, indem die Komponenten in einem Rührwerksbehälter vorgemischt werden. In diesem fertigen Lösungsmittelsystem werden dann die jeweiligen pharmazeutischen Wirkstoffe gelöst. Diese Lösung wird vorzugsweise filtriert und entlüftet und dann in üblicher Weise in Weichgelatinkapseln eingetragen. Vorzugsweise kommt hierbei das Rotazy-Die-Verfahren zur Anwendung.

Die Erfindung wird durch die nachfolgenden Beispiele und Vergleichsversuche näher erläutert.

| Beispiel 1 (Placebolösung) | |
|---|---|
| Benzylalkohol | 5 % |
| Glycerol | 5 % |
| mittelkettige Partialglyceride (Inwitor 988)^{R} | 80 % |
| Tween 80^{R} | 10 % |

Diese Mischung wird in an sich bekannter Weise zu Weichgelatinekapseln weiterverarbeitet. Die Kapseln sind physikalisch stabil. In Kontakt mit Gastrointestinalflüssigkeit sind sie selbstemulgierend.

| Vergleichsbeispiel 1 | |
|---|---|
| Benzylalkohol | 5 % |
| Glycerol | 5 % |
| mittelkettige Partialglyceride (Inwitor 988)^{R} | 35 % |
| Tween 80^{R} | 55 % |

Das Gemisch dieser Komponenten ergab bei der Einfüllung in Weichgelatinekapseln gravierende Produktionsprobleme. Es entstanden offene Kapseln infolge ungenügender Nahtbildung. In Kontakt mit wenig Wasser entsteht nur eine grobe Emulsion.

| Beispiel 2 | |
|---|---|
| Dextromethorphan | 10 % |
| Polyethylenglykol 400 | 6 % |
| Ethanol | 4 % |
| Tegomuls^{R} | 75 % |
| Cremophor RH 40^{R} | 5 % |

Diese Mischung ließ sich in an sich bekannter Weise zu Weichgelatinekapseln verarbeiten. Es resultierten physikalisch stabile Kapseln, die in Kontakt mit Gastrointestinalflüssigkeit selbstemulgierend sind.

| Vergleichsbeispiel 2 | |
|---|---|
| Dextromethorphan | 10 % |
| Polyethylenglykol 400 | 6 % |
| Ethanol | 30 % |
| Tegomuls^{R} | 44 % |
| Cremophor RH 40^{R} | 10 % |

Bereits bei der Produktion von Weichgelatinekapseln aus dieser Lösung kam es zu einem Verlust von 20 % der eingesetzten Ethanolmenge. Bei der Trocknung der Weichgelatinekapseln wurde der Verlust von weiteren 35 % des eingesetzten Ethanols festgestellt.

Diese Kapseln mußten in Aluminium-Blister verpackt werden, um weiteren Gewichtsverlust zu vermeiden. Diese Verpackung gestattet es nicht, die Kapseln dem Verbraucher in der Weise anzubieten, daß er ohne Öffnung der Packung die klare transparente Wirkstofflösung sehen kann.

| Beispiel 3 | |
|---|---|
| Indometacin | 5 % |
| Inwitor 742^{R} | 78 % |
| Tagat O₂ | 7 % |
| Propandiol | 10 % |

Diese Mischung ließ sich problemlos zu Weichgelatinekapseln weiterverarbeiten. In Kontakt mit Gastrointestinalflüssigkeit kam es zur Ausbildung eines tyndallisierenden Systems.

| Vergleichsbeispiel 3 | |
|---|---|
| Indometacin | 5 % |
| Inwitor 742^{R} | 43 % |
| Tagat O₂ | 20 % |
| Propandiol | 15 % |
| Wasser | 17 % |

Bei der Herstellung von Weichgelatinekapseln wurde nach der Trocknung eine massive Deformation insbesondere im Bereich der Kapselnaht festgestellt. Dies ist erklärbar dadurch, daß das im Füllgut enthaltende Wasser herausgetrocknet wird, so daß es zu einer Volumenkonzentration kommt, in deren Folge die Kapselhülle sich deformiert.

Bei der Abfüllung dieser Zubereitung in Hartgelatinekapseln kam es durch den Wasseranteil zunächst zu einem Erweichen der Kapselhülle, in deren Folge bei einem anschließenden Trocknungsprozeß die Kapseln entweder stark deformieren oder die Kapselwand sogar aufbricht und Füllgut austritt.

## Patentansprüche

1. Pharmazeutische Präparate zur oralen Anwendung in flüssig befüllten Weichgelatinekapseln, **dadurch gekennzeichnet, dass** der Wirkstoff gelöst vorliegt in einem einphasigen System, enthaltend jeweils bezogen auf das wirkstofffreie System 1 bis 20 Gew.-% Polyethylenglycole und/oder Benzylalkohol, 1 bis 20 Gew.-% eines oder mehrerer Tenside, 75 bis 98 Gew.-% eines oder mehrerer Co-Tenside und weniger als 5 Gew.-% Ethanol und weniger als 10 Gew.-% Wasser.

2. Pharmazeutische Präparate gemäß Anspruch 1, **dadurch gekennzeichnet, dass** der Gehalt an Polyethylenglycol 2,5 bis 12,5 Gew.-% beträgt.

3. Pharmazeutische Präparate gemäß Anspruch 1 oder 2, **dadurch gekennzeichnet, dass** der Gehalt an Tensiden 5 bis 15 Gew. -% beträgt.

4. Pharmazeutische Präparate gemäß einem der Ansprüche 1 bis 3, **dadurch gekennzeichnet, dass** der Gehalt an Co-Tensiden 79 bis 90 Gew.-% beträgt.

5. Verfahren zur Herstellung von pharmazeutischen Präparaten zur oralen Anwendung in flüssig befüllten Weichgelatinekapseln, **dadurch gekennzeichnet, dass** der Wirkstoff gelöst wird in einem einphasigen System, enthaltend jeweils bezogen auf das wirkstofffreie System 1 bis 20 Gew.-% Polyethylenglycole und/oder Benzylalkohol, 1 bis 20 Gew.-% eines oder mehrerer Tenside, 75 bis 98 Gew.-% eines oder mehrerer Co-Tenside und weniger als 5 Gew.-% Ethanol und weniger als 10 Gew.-% Wasser, und in an sich bekannter Weise in Gelatinekapseln abgefüllt wird.

6. Verfahren gemäß Anspruch 5, **dadurch gekennzeichnet, dass** der Gehalt an Polyethylenglycolen 2,5 bis 12,5 Gew.-% beträgt.

7. Verfahren gemäß Anspruch 5 oder 6, **dadurch gekennzeichnet, dass** der Gehalt an Tensiden 5 bis 15 Gew.-% beträgt.

8. Verfahren gemäß einem der Ansprüche 5 bis 7, **dadurch gekennzeichnet, dass** der Gehalt an Co-Tensiden 79 bis 90 Gew.-% beträgt.

## Claims

1. Pharmaceutical preparations for oral application in liquid-filled soft gelatin capsules, **characterized in that** the active ingredient is dissolved in a one-phase system containing, respectively based on the system without the active ingredient, from 1 to 20% by weight of polyethylene glycols and/or benzyl alcohol, from 1 to 20% by weight of one or more surfactants, from 75 to 98% by weight of one or more co-surfactants, and less than 5% by weight of ethanol and less than 10% by weight of water.

2. The pharmaceutical preparations according to claim 1, **characterized in that** their content of polyethylene glycol is from 2.5 to 12.5% by weight.

3. The pharmaceutical preparations according to claim 1 or 2, **characterized in that** their content of surfactants is from 5 to 15% by weight.

4. The pharmaceutical preparations according to any of claims 1 to 3, **characterized in that** their content of co-surfactants is from 79 to 90% by weight.

5. A process for making pharmaceutical preparations for oral application in liquid-filled soft gelatin capsules, **characterized in that** the active ingredient is dissolved in a one-phase system containing, respectively based on the system without the active ingredient, from 1 to 20% by weight of polyethylene glycols and/or benzyl alcohol, from 1 to 20% by weight of one or more surfactants, from 75 to 98% by weight of one or more co-surfactants, and less than 5% by weight of ethanol and less than 10% by weight of water, and the mixture is filled in gelatin capsules in a per se known manner.

6. The process according to claim 5, **characterized in that** the content of polyethylene glycols is from 2.5 to 12.5% by weight.

7. The process according to claim 5 or 6, **characterized in that** the content of surfactants is from 5 to 15% by weight.

8. The process according to any of claims 5 to 7, **characterized in that** the content of co-surfactants is from 79 to 90% by weight.

## Revendications

1. Préparations pharmaceutiques à usage oral dans des capsules de gélatine molle remplies de liquide, **caractérisées en ce que** le principe actif est présent à l'état dissous dans un système à une phase, contenant, par rapport au système exempt de principe actif, 1 à 20 % en poids de polyéthylèneglycols et/ou d'alcool benzylique, 1 à 20 % en poids d'un ou plusieurs agents tensio-actifs, 75 à 98 % en poids d'un ou plusieurs co-agents tensio-actifs et moins de 5 % en poids d'éthanol et moins de 10 % en poids d'eau.

2. Préparations pharmaceutiques selon la revendication 1, **caractérisées en ce que** la teneur en polyéthylèneglycol est de 2,5 à 12,5 % en poids.

3. Préparations pharmaceutiques selon la revendication 1 ou 2, **caractérisées en ce que** la teneur en agents tensio-actifs est de 5 à 15 % en poids.

4. Préparations pharmaceutiques selon l'une des revendications 1 à 3, **caractérisées en ce que** la teneur en co-agents tensio-actifs est de 79 à 90 % en poids.

5. Procédé de fabrication de préparations pharmaceutiques à usage oral dans des capsules de gélatine molle remplies de liquide, **caractérisé en ce que** le principe actif est dissous dans un système à une phase, contenant, par rapport au système exempt de principe actif, 1 à 20 % en poids de polyéthylèneglycols et/ou d'alcool benzylique, 1 à 20 % en poids d'un ou plusieurs agents tensio-actifs, 75 à 98 % en poids d'un ou plusieurs co-agents tensio-actifs et moins de 5 % en poids d'éthanol et moins de la % en poids d'eau, et est introduit dans des capsules de gélatine d'une manière connue en soi.

6. Procédé selon la revendication 5, **caractérisé en ce que** la teneur en polyéthylèneglycols est de 2,5 à 12,5 % en poids.

7. Procédé selon la revendication 5 ou 6, **caractérisé en ce que** la teneur en agents tensio-actifs est de 5 à 15 % en poids.

8. Procédé selon l'une des revendications 5 à 7, **caractérisé en ce que** la teneur en co-agents tensio-actifs est de 79 à 90 % en poids.
